# EUROPEAN PATENT APPLICATION

(11) **EP 2 216 397 A1**
(43) Date of publication of application: **11.08.2010**
(21) Application number: 08857364.7
(22) Date of filing: 03.12.2008
(51) Int. Cl.: C12M 3/00, C12M 1/00

(54) **CELL PROCESSING DEVICE**

(30) Priority: 04.12.2007 JP 2007313543
(71) Applicant: Olympus Corporation, Shibuya-ku Tokyo 151-0072 (JP)
(72) Inventor: TAMURA, Tomoaki, Tokyo 151-0072 (JP); WATANABE, Sadahiro, Tokyo 151-0072 (JP); TOKUDA, Kazunari, Tokyo 151-0072 (JP)
(74) Representative: Schicker, Silvia
(86) International application number: PCT/JP2008/071961
(87) International publication number: WO 2009/072520

(57) **Abstract**

In a closed system having a large number of containers connected via pipes, a leakage of a liquid from the closed system can be more reliably detected by a convenient and inexpensive method. Provided is a cell processing device (1) comprising a plurality of processing containers (2a and 10) for processing cells, processing liquid containers (4a) for containing a processing liquid to be supplied to the processing containers (2a and 10), waste liquid containers (5a) for containing a waste liquid which has been discharged from the processing containers (2a and 10) after processing the cells in the processing containers (2a and 10), a plurality of pipe lines (14) for connecting these containers (2a, 4a, 5a, and 10), a pump (15) for supplying the processing liquid and the waste liquid via the pipe lines (14), weight meters (2b, 4b, and 5b) for weighing the processing container (2a), the waste liquid containers (5a), and/or the processing liquid containers (4a), and a leakage determination section (18) for determining the occurrence of a leakage of the processing liquid or the waste liquid based on outputs from the weight meters (2b, 4b, and 5b).

## Description

### Technical Field

The present invention relates to a cell processing device.

### Background Art

Heretofore, there is a known cell processing device for digesting a biological tissue such as an adipose tissue by agitation with a digestive enzyme liquid, and for collecting adipose-derived cells by concentrating the thus yielded cell suspension with a centrifugal separator (for example, refer to Patent Citation 1).
In this cell processing device, a washing process is performed by supplying the biological tissue with a processing liquid such as a washing liquid, and after this process the washing liquid is discharged as a waste liquid. In addition, the cell suspension yielded by digestion of the biological tissue is also supplied with a processing liquid such as the washing liquid and is subjected to a concentrating process in the centrifugal separator, and after this process the supernatant is discharged as a waste liquid.
Moreover, a blood processing system having a spill sensor is also disclosed (for example, refer to Patent Citation 2).
Patent Citation 1:
   PCT International Publication No. WO 2005/012480A2 Pamphlet
Patent Citation 2
   Japanese Translation of PCT International Application, Publication No. Hei 9-502914

### Disclosure of Invention

However, in the cell processing device of Patent Citation 1, the aforementioned various processes are not performed in one area, but respectively performed in a plurality of processing containers. Therefore, the respective processing containers and the respective processing liquid containers have to be connected by a great number of conduits. For this reason, problems such as a leakage of a processing liquid or a waste liquid from a processing container, a conduit, and so forth, are likely to occur with high possibility. In particular, a leakage of a waste liquid may cause pollution of the external environment, and therefore it is necessary to more reliably prevent a leakage of a waste liquid which contains a biological tissue to the outside.
In addition, the spill sensor of Patent Citation 2 may lead to an increase of the overall scale if all processing containers and conduits are to be equipped with it, and involves a concern in that the detection may be difficult for some kind of leakage occurring without touching an electrode.

The present invention was made to address the above-mentioned situations with an object of providing a cell processing device, with which a leakage of a liquid from a closed system having a large number of containers connected via pipes can be more reliably detected by a convenient and inexpensive method.

In order to achieve the above-mentioned object, the present invention provides the following solutions.
One aspect of the present invention is a cell processing device comprising: a plurality of processing containers for processing cells; a processing liquid container for containing a processing liquid to be supplied to the processing containers; a waste liquid container for containing a waste liquid which has been discharged from the processing containers after processing the cells in the processing containers; a plurality of pipe lines for connecting these containers; a pump for supplying the processing liquid and the waste liquid via the pipe lines; weight meters for weighing the processing containers, the waste liquid container, and/or the processing liquid container; and a leakage determination section for determining the occurrence of a leakage of the processing liquid or the waste liquid based on outputs from the weight meters.

According to the above-mentioned aspect, cells are contained in the processing containers and the processing liquid in the processing liquid container is driven by the pump to be supplied into the processing containers, by which these cells are processed in the processing containers. After this process, a processed waste liquid is produced in the processing containers, and thus the waste liquid is discharged from the processing containers into the waste liquid container, by which the processed cells can be obtained.
In this case, since the processing containers, the processing liquid container, the waste liquid container, and the pipes for connecting them constitute a closed system, the weight balance of this system should be zero if there is no liquid leakage. Therefore, it becomes possible to more reliably detect the occurrence of a leakage and the position of the leakage by weighing the processing containers, the processing liquid container, and the waste liquid container by the weight meters so as to know if their weights are balanced or make any difference between before and after the processing liquid or the waste liquid has been supplied or discharged into or from the respective containers.

In the above-mentioned aspect, the leakage determination section may also determine the occurrence of a leakage by comparing a difference in outputs from the weight meters between before and after the waste liquid has been discharged from the processing containers, with a difference in outputs from the weight meters between before and after the waste liquid has flown into the waste liquid container.
By so doing, it becomes possible to more reliably detect a leakage of the waste liquid occurring during the time when the waste liquid is being discharged from the processing containers into the waste liquid container.

For example, assuming a case where a mixture solution having a biological tissue and a liquid is supplied into the processing containers and then the amount of the biological tissue contained therein is to be detected, it is necessary to discharge all liquid in the mixture solution into the waste liquid container as a waste liquid. In this case, the difference between the weight of the processing containers containing the mixture solution and the weight of the processing containers after the waste liquid has been discharged is compared with the difference in the weight of the waste liquid container between before and after the waste liquid has flown therein. By so doing, if these values are remarkably different, it can be determined that a leakage of a liquid occurs in any point of the processing containers, the waste liquid container, and pipes therebetween.

In addition, in the above-mentioned aspect, the leakage determination section may also determine the occurrence of a leakage by comparing a difference in outputs from the weight meters between before and after the processing liquid has been supplied from the processing liquid container, with a difference in the outputs from the weight meters between before and after the waste liquid has flown into the waste liquid container.
By so doing, it becomes possible to more reliably detect a leakage occurring during the time when the processing liquid or the waste liquid is being transferred from the processing liquid container into the processing containers, or from the processing containers into the waste liquid container.

For example, assuming a case where the processing liquid is supplied from the processing liquid container into the processing containers and the processing is performed in the processing containers, and then after the process the waste liquid is discharged from the processing containers into the waste liquid container; the difference in the weight of the processing liquid container between before and after the processing liquid has been supplied from the processing liquid container is compared with the difference in the weight of the waste liquid container between before and after the waste liquid has flown into the waste liquid container, and if these values are remarkably different, it can be determined that a leakage of a liquid occurs in any point of the processing liquid container, the processing containers, the waste liquid container, and pipes therebetween.

The present invention offers an effect in which a leakage of a liquid from a closed system having a large number of containers connected via pipes can be more reliably detected by a convenient and inexpensive method.

### Brief Description of Drawings

[FIG. 1] FIG. 1 is an overall block diagram showing a cell processing device according to one embodiment of the present invention.
[FIG. 2] FIG. 2 is an overall block diagram showing a flow of a liquid from the decomposition processing section into the waste liquid collecting section in the cell processing device of FIG. 1.
[FIG. 3] FIG. 3 is an overall block diagram showing a flow of a washing liquid from the washing liquid containing section into the cell processing section in the cell processing device of FIG. 1.
[FIG. 4] FIG. 4 is an overall block diagram showing a flow of a cell suspension from the decomposition processing section into the cell concentrating section in the cell processing device of FIG. 1.
[FIG. 5] FIG. 5 is an overall block diagram which illustrates the manner of supply of the cell suspension into the sample port in the cell processing device of FIG. 1.
[FIG. 6] FIG. 6 is an overall block diagram which illustrates the manner of collection of supernatants that have been separated in the cell concentrating section, into the waste liquid collecting section in the cell processing device of FIG. 1.
[FIG. 7] FIG. 7 is an overall block diagram which illustrates the manner of supply of the washing liquid to adipose-derived cells that has been concentrated in the cell concentrating section in the cell processing device of FIG. 1.
[FIG. 8] FIG. 8 shows a modified example of the centrifuge container in the cell processing device of FIG. 1.

### Explanation of Reference:

- 1:: Cell processing device
- 2a:: Decomposition processing container (processing container)
- 2b, 4b, 5b:: Weight meters
- 4a:: Washing liquid containers (processing liquid containers)
- 5a:: Waste liquid containers
- 10:: Centrifuge containers (processing containers)
- 14:: Tube (pipe line)
- 15:: Pump
- 18:: Leakage determination section

### Best Mode for Carrying Out the Invention

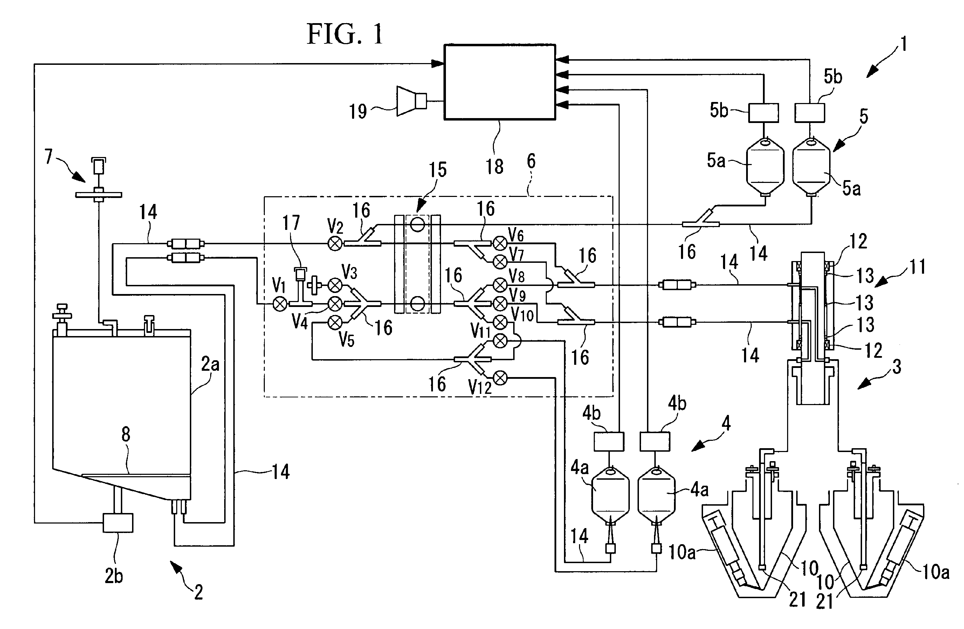

Hereunder is a description of the cell processing device 1 according to one embodiment of the present invention, with reference to FIG. 1 to FIG. 7.
As shown in FIG. 1, the cell processing device 1 according to this embodiment comprises: a decomposition processing section 2 for containing and agitating a biological tissue such as an adipose tissue with a digestive enzyme; a cell concentrating section 3 for concentrating a cell suspension that has been yielded by isolation of adipose-derived cells from the adipose tissue as a result of the decomposition process in the decomposition processing section 2; a washing liquid containing section 4 for storing a washing liquid to wash the adipose-derived cells that have been concentrated in the cell concentrating section 3; a waste liquid collecting section 5 for storing a waste liquid that has been discharged from the concentrating process (including the washing process) in the cell concentrating section 3; and transfer lines (pipes, cell transfer section) 6 for connecting them.

The decomposition processing section 2 is equipped with a decomposition processing container 2a. The decomposition processing container 2a comprises an introduction section 7 for introducing an adipose tissue sampled from a human into the decomposition processing section 2, and a filter 8 for allowing the transmission of adipose-derived cells isolated from the adipose tissue but blocking the transmission of the adipose tissue. In addition, the decomposition processing container 2a is equipped with a weight meter 2b for weighing the decomposition processing container 2a.

In the example shown in this drawing, the cell concentrating section 3 comprises two centrifuge containers 10 and a centrifugal separator 11 (only the joint part is shown in the drawing) for rotating the centrifuge containers 10 while orienting their bottoms toward the radially outward.

The reference sign 12 denotes a bearing, the reference sign 13 denotes a sealing member, and the reference sign 21 denotes a supernatant suction part for sucking a supernatant. The centrifuge container 10 is integrally provided with a syringe 10a for collecting the finally concentrated adipose-derived cells.
The washing liquid containing section 4 comprises washing liquid containers (processing liquid containers) 4a for containing a washing liquid and weight meters 4b for weighing the washing liquid containers 4a. In addition, the waste liquid collecting section 5 comprises waste liquid containers 5a for containing a waste liquid and weight meters 5b for weighing the waste liquid containers 5a.

The cell transfer lines 6 comprise: a plurality of tubes 14 for connecting the decomposition processing section 2, the cell concentrating section 3, the washing liquid containing section 4, and the waste liquid collecting section 5; a pump 15 for pressing the tubes 14 from the outside so as to send out the internal liquid; joints 16 for making branches of the tubes 14; a plurality of valves V₁ to V₁₂ for opening and closing the tubes 14 in respective positions; and a sample port 17 which is located in a midpoint of the tube 14 and is capable of portioning out the cell suspension.

Moreover, the outputs from the respective weight meters 2b, 4b, and 5b are connected to a leakage determination section 18. The leakage determination section 18 is equipped with a speaker 19 for announcing the determination result.
The leakage determination section 18 is designed to determine whether or not a leakage occurs in each section based on the outputs from the respective weight meters 2b, 4b, and 5b. If the leakage determination section 18 determines that a leakage occurs, this determination is announced to the outside through the speaker 19.

Hereunder is a description of the operation of the cell processing device 1 according to the thus constructed embodiment.
In order to decompose a biological tissue, for example, an adipose tissue, and to collect cell-derived cells with use of the cell processing device 1 according to this embodiment, firstly, the amount of the adipose tissue supplied through the introduction section 7 into the decomposition processing container 2a of the decomposition processing section 2 is detected.

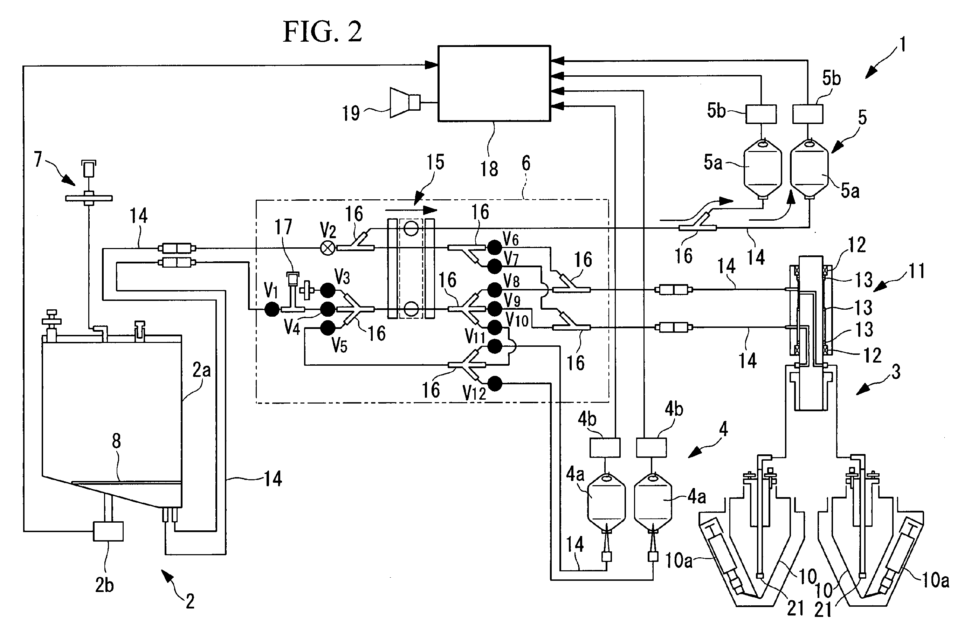

At the beginning, the weight W₁ of the decomposition processing container 2a before the introduction of the adipose tissue is measured by the weight meter 2b. The adipose tissue is introduced in a mixed state with a body fluid or physiological saline from the introduction section 7. Therefore, the weight W₂ of the decomposition processing container 2a after the introduction of the adipose tissue is measured by the weight meter 2b, and then, as shown by solid circles in FIG. 2, the valves V₁ and V₃ to V₁₂ except for the valve V₂ are closed and the pump 15 is turned to the normal direction in the cell transfer section 6, by which the weight W₃ after discharging the liquid from the decomposition processing container 2a is also measured by the weight meter 2b.

The weight W of the adipose tissue can be calculated by the equation of W = W₃ - W₁.
In addition, before discharging the liquid from the decomposition processing container 2a, the weight W₄ of the waste liquid containers 5a is measured by the weight meters 5b. Then, after discharging the liquid into the waste liquid containers 5a, the weight W₅ of the waste liquid containers 5a is measured by the weight meters 5b.

In the leakage determination section, the weight W₆ of the liquid discharged from the decomposition processing container 2a is calculated by the equation of W₆ = W₂ - W₃, while the weight W₇ of the liquid discharged into the waste liquid containers 5a is calculated by the equation of W₇ = W₅ - W₄. Then, a comparison is made between the weight W₆ and the weight W₇. If the ratio therebetween ratio of W₇ to W₆ is equal to or lower than a predetermined threshold, the leakage determination section determines that a leakage of the liquid occurs and announces the determination to the outside through the speaker 19. If the ratio between the weights W₆ and W₇ is greater than the predetermined threshold and is close to 1, the leakage determination section determines that there is no occurrence of a leakage of the liquid.

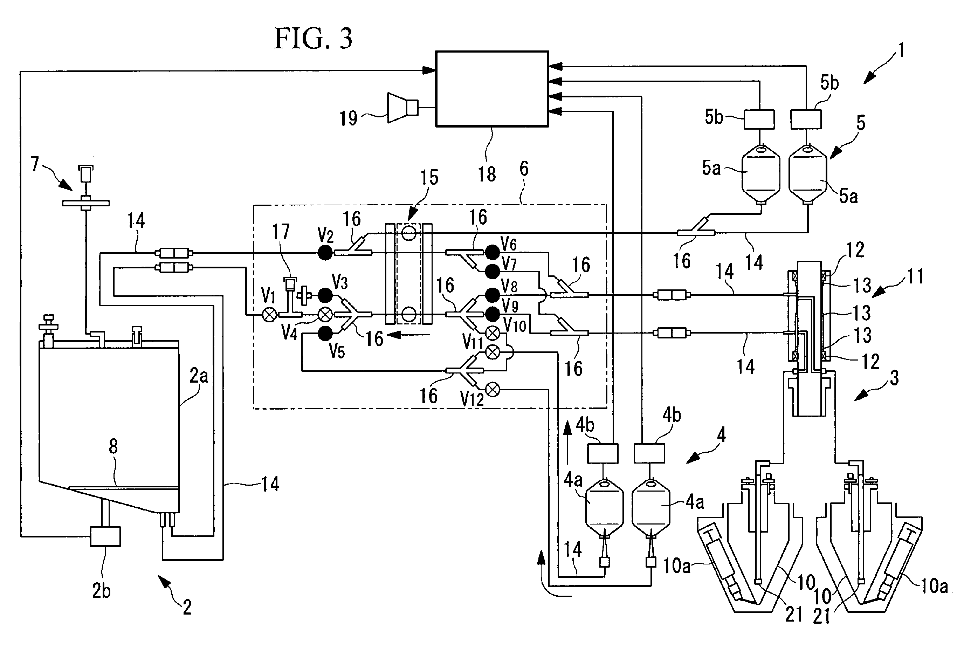

Next, the cell processing device 1 performs a washing process on the adipose tissue.
In the washing process on the adipose tissue, as shown in FIG. 3, the valves V₂, V₃, and V₅ to V₉ are closed, the valves V₁, V₄, and V₁₀ to V₁₂ are opened, and the pump 15 is turned to the inverted direction in the cell transfer section 6. By so doing, the washing liquid stored in the washing liquid containers 4a of the washing liquid containing section 4 is supplied to the decomposition processing container 2a. Then, the washing liquid and the adipose tissue are agitated in the decomposition processing container 2a to thereby wash out the body fluid or the like adhering to the adipose tissue.

Thereafter, again, as shown in FIG. 2, the valves V₁ and V₃ to V₁₂ except for the valve V₂ are closed and the pump 15 is turned to the normal direction in the cell transfer section 6, by which the washing liquid in the decomposition processing container 2a of the decomposition processing section 2 is sucked and discharged into the waste liquid containers 5a by the pump 15.
In this case, the weights W₈ and W₉ of the washing liquid containers 4a before and after supplying the washing liquid to the decomposition processing container 2a and the weights W₁₀ and W₁₁ of the waste liquid containers 5a before and after discharging the washing liquid into the waste liquid containers 5a are measured.

In the leakage determination section 18, the weight W₁₂ of the washing liquid supplied from the washing liquid containers 4a is calculated by the equation of W₁₂ = W₈ - W₉, while the weight W₁₃ of the washing liquid discharged into the waste liquid containers 5a after the washing process is calculated by the equation of W₁₃ = W₁₁ - W₁₀. Then, a comparison is made between the weight W₁₃ and the weight W₁₂. If the ratio of W₁₃ to W₁₂ is equal to or lower than a predetermined threshold, the leakage determination section 18 determines that a leakage of the washing liquid occurs and announces the determination to the outside through the speaker 19. If the ratio between the weights W₁₂ and W₁₃ is greater than the predetermined threshold and is close to 1, the leakage determination section 18 determines that there is no occurrence of a leakage of the washing liquid.

Next, the cell processing device 1 supplies a digestive enzyme liquid at an amount which has been determined according to the thus detected weight W of the adipose tissue, into the decomposition processing container 2a of the decomposition processing section 2, and agitates the mixture under heating. By so doing, the adipose tissue is decomposed by the digestive enzyme to thereby yield a cell suspension in which adipose-derived cells are isolated from the adipose tissue.

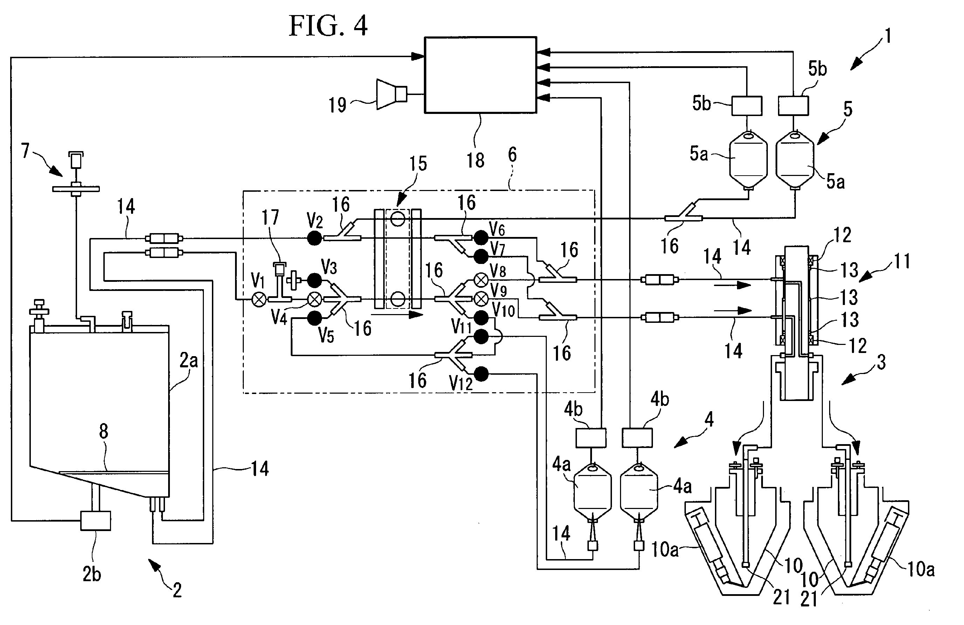

When a predetermined amount of the cell suspension is obtained, as shown in FIG. 4, the valves V₂, V₃, V₅ to V₇, and V₁₀ to V₁₂ are closed, the valves V₁, V₄, V₈, and V₉ are opened, and the pump 15 is turned to the normal direction in the cell transfer section 6. By so doing, the cell suspension in the decomposition processing container 2a of the decomposition processing section 2 is sucked and supplied into the cell concentrating section 3 by the pump 15.

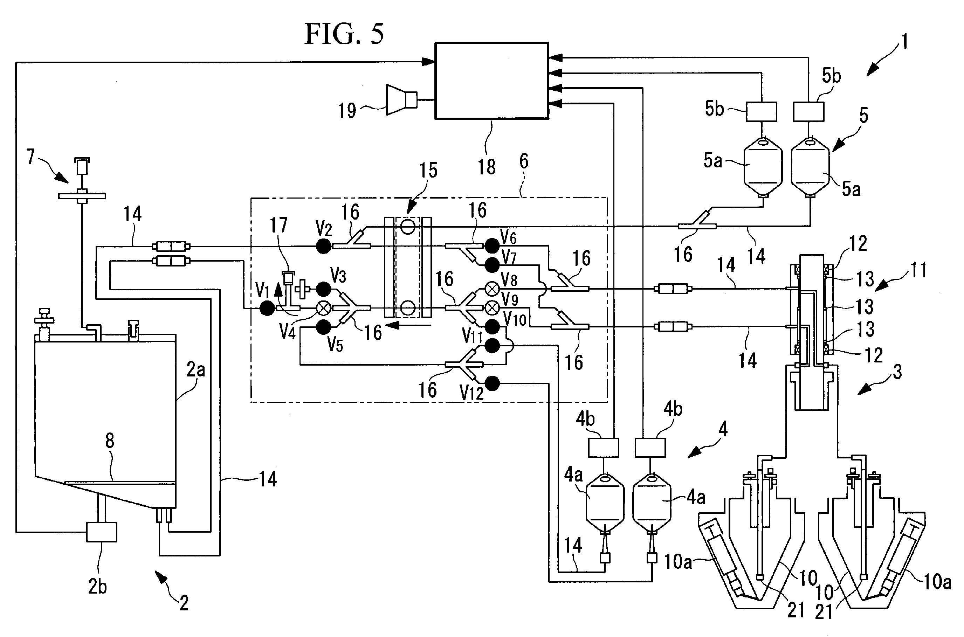

Here, before all the cell suspension is sent to the cell concentrating section 3, as shown in FIG. 5, the valve V₁ is closed and the pump 15 is turned to the inverted direction. By so doing, the cell suspension in the tube 14 can flow backward to be sent to the sample port 17. Also, by attaching a syringe to the sample port 17 and sucking up the cell suspension, the cell suspension in the sample port 17 can be collected into the syringe. The cell suspension collected in the syringe is sent to an examination device (not shown) in which, for example, an ATP assay and an LDH assay are to be performed.

The ATP assay is to measure the ATP level by adding an ATP assay reagent (manufactured by Kikkoman) to the given cell suspension. Since the ATP level is proportional to the number of cells, it is possible to measure the number of cells in the cell suspension. In addition, since bacterial or microbial contamination brings about an extreme increase in the ATP level, it is also possible to evaluate the bacterial or such contamination.

Moreover, the LDH assay is to measure the LDH level by adding an LDH assay reagent (manufactured by Wako Pure Chemical Industries) to the given cell suspension. Since the LDH level increases in response to a stress on cells such as an injury, it is possible to measure the stress level of cells. It is known that a normal cell suspension shows an LDH level of 100 IU/L/37°C. In addition, it is also possible to measure the number of cells (viability determination) by using an LDH activity-based cytotoxicity assay reagent (for example, the LDH-cytotoxic test kit manufactured by Wako Pure Chemical Industries).

Thereafter, again, as shown in FIG. 4, the valve V₁ is opened and the pump 15 is turned to the normal direction to send a sufficient amount of the cell suspension to the cell concentrating section 3. Then, in the cell concentrating section 3, the cell suspension is charged in the centrifuge containers 10 and subjected to a centrifugal process through the operation of the centrifugal separator 11. By so doing, the adipose-derived cells having a relatively large density in the cell suspension are collected to the bottoms of the centrifuge containers 10.

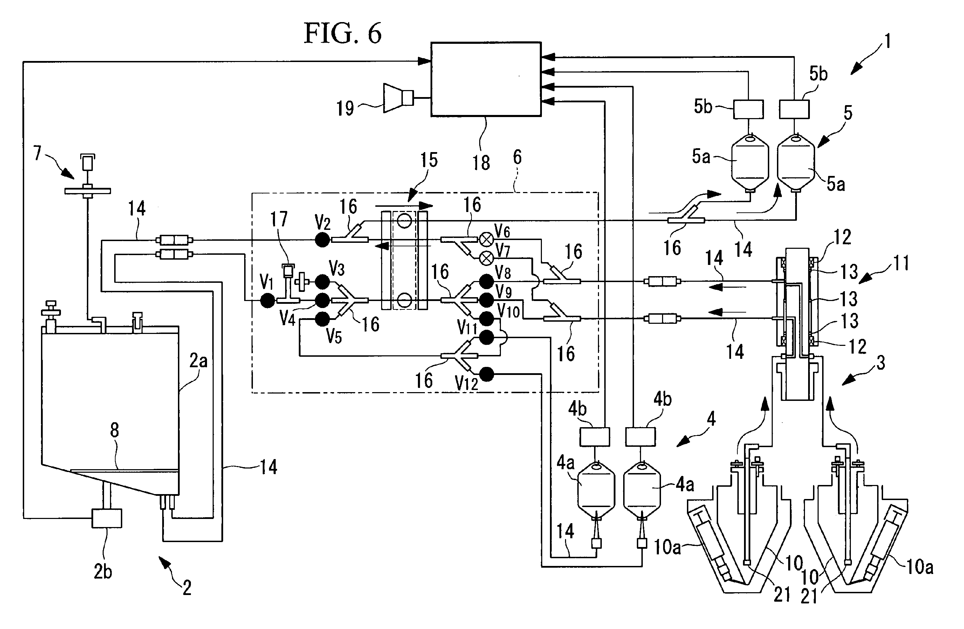

In this state, as shown in FIG. 6, the valves V₁ to V₅ and V₈ to V₁₀ are closed, the valves V₆ and V₇ are opened, and the pump 15 is turned to the inverted direction in the cell transfer section 6. By so doing, the supernatants produced in the centrifuge containers 10 are sucked and sent to the waste liquid containers 5a of the waste liquid collecting section 5. By sufficiently sending the supernatants to the waste liquid containers 5a, pellets of the adipose-derived cells and predetermined tiny amounts of supernatants are left in the centrifuge containers 10. The liquid level of the supernatant at this time is previously set by the position of the suction end.

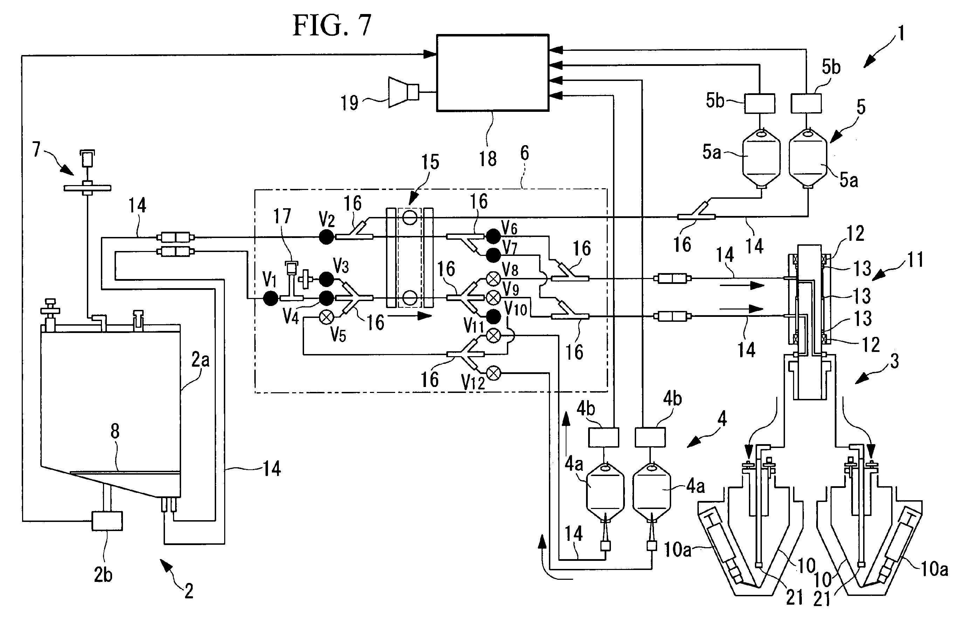

Thereafter, as shown in FIG. 7, the valves V₁ to V₄, V₆, V₇, and V₁₀ are closed, the valves V₅, V₈, V₉, V₁₁, and V₁₂ are opened, and the pump 15 is turned to the normal direction. By so doing, the washing liquid stored in the washing liquid containers 4a of the washing liquid containing section 4 is supplied into the centrifuge containers 10 to be mixed and resuspended with the adipose-derived cells and the supernatants contained in the centrifuge containers 10.

Then, the step of supplying the washing liquid and resuspending the mixture, the step of collecting the adipose-derived cells to the bottoms of the centrifuge containers 10 through the operation of the centrifugal separator 11, and the step of sucking the supernatants and disposing them into the waste liquid containers 5a of the waste liquid collecting section 5, are repeated to thereby dilute the digestive enzyme in the cell suspension to a degree of concentration that would not damage the adipose-derived cells.

In this case, per each repetition of the steps of supplying the washing liquid, performing the centrifugal process, and discharging the supernatants, in a state where the supernatants are sucked, the weight W₁₄ of the processing liquid containers 4a before supplying the washing liquid into the centrifuge containers 10 is measured by the weight meters 4b. In addition, the weight W₁₅ of the waste liquid containers 5a is also measured by the weight meters 5b.

Then, at the time after a predetermined amount of the washing liquid has been supplied from the washing liquid containers 4a into the centrifuge containers 10, the weight W₁₆ of the washing liquid containers 4a is measured by the weight meters 4b. In addition, at the time after the centrifuged supernatants have been sucked down to a predetermined level and discharged into the waste liquid containers 5a, the weight W₁₇ of the waste liquid containers 5a is measured by the weight meters 5b.

In the leakage determination section 18, the weight W₁₈ of the washing liquid supplied from the washing liquid containers 4a into the centrifuge containers 10 is calculated by the difference between the weights W₁₄ and W₁₅ of the washing liquid containers 4a before and after the supply of the washing liquid, that is, the equation of W₁₈ = W₁₄ - W₁₅ . Meanwhile, the weight W₁₉ of the supernatants discharged into the waste liquid containers 5a is calculated by the equation of W₁₉ = W₁₇ - W₁₆.

Then, a comparison is made between the weight W₁₈ and the weight W₁₉. If the ratio of W₁₉ to W₁₈ is equal to or lower than a predetermined threshold, the leakage determination section 18 determines that a leakage of the liquid occurs and announces the determination to the outside through the speaker 19. If the ratio between the weights W₁₈ and W₁₉ is greater than the predetermined threshold and is close to 1, the leakage determination section 18 determines that there is no occurrence of a leakage of the liquid.

Next, the adipose-derived cells sedimented at the bottoms of the centrifuge containers 10 are sucked by the syringes 10a that are integrally equipped in the centrifuge containers 10, and then these syringes 10a are detached from the centrifuge containers 10. By so doing, the adipose-derived cells can be directly transferred to be implanted into the diseased part.

In this manner, according to the cell processing device 1 of this embodiment, a leakage of a liquid such as a washing liquid in the processing containers 2a, 4a, and 5a, and the cell transfer section 6 can be detected only by weighing the various processing containers (decomposition processing container 2a, washing liquid containers 4a, and waste liquid containers 5a) and making comparisons therebetween. Accordingly, the advantage is that a leakage of a liquid can be more reliably detected without a need of providing a large number of liquid leakage sensors at respective positions of pipes.

As a result, particularly, it becomes possible to more reliably prevent a leakage of a waste liquid which contains a biological tissue after processing an adipose tissue or adipose-derived cells to the outside, and therefore to prevent pollution of the external environment and other undesirable situations such as cross contamination when another biological tissue is processed.
In addition, since a leakage of a liquid can be detected by a small number of weight meters 2b, 4b, and 5b, advantageously the structure can be made at low cost.

Moreover, according to the cell processing device 1 of this embodiment, the adipose-derived cells are not examined at the time of the final collection but examined at the time of collection from the sample port 17 located in a midpoint during the transfer from the decomposition processing section 2 to the cell concentrating section 3. Therefore, it becomes possible to examine the condition of the adipose-derived cells during the time when they are concentrated and collected into the syringes 10a in the cell concentrating section 3. As a result, an advantage is provided in which the concentrated adipose-derived cells can be implanted immediately after the concentration process.

Furthermore, as a result of examining of the cell suspension collected from the sample port 17, if the number of cells is found to be small, the examination result can be displayed on a display section (not shown) to give feedback to the operator so that he/she can perform the decomposition process of cells again in the decomposition processing section 2, or such an operation. In addition, as a result of examining of the cell suspension collected from the sample port 17, if an undesirable situation such as a highly stressed state of cells, bacterial infection, or the like, is found, the undergoing concentrating process on cells can be stopped. By so doing, wasteful processes can be prevented beforehand.

In this embodiment, the position of the sample port 17 is located between the valve V₁ and the pump 15 so that the cell suspension can be supplied to the sample port 17 by turning the pump 15 to the inverted direction. However, instead of this, the sample port 17 may also be located somewhere else.
Moreover, the cell suspension supplied to the sample port 17 can be taken out by a detachable syringe. However, instead of this, an examination device (cell examination section) may be connected to the sample port 17 so that the cell suspension supplied to the sample port 17 can be directly supplied to the examination device to examine the condition of cells. By so doing, the condition of cells can be examined without an operator, and it can be automated to perform an additional decomposition process or to stop the concentrating process in the decomposition processing section, and to perform such an operation, in accordance with the result of examining.

Furthermore, the cell suspension taken out from the syringe is transferred to the examination device for examining. However, instead of this, a syringe storing a reagent for measuring each item therein, or a syringe having reagent components coated on its inner wall may be used so that the examination can be promptly carried out simply by sucking the cell suspension into the syringe.

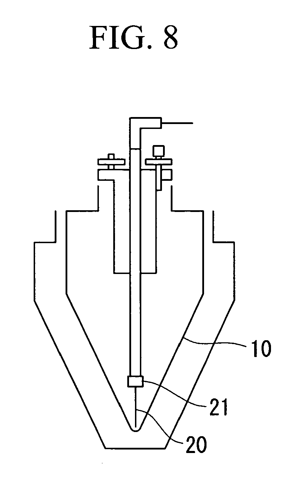

In addition, this embodiment is illustrated by the example in which the centrifuge container 10 of the cell concentrating section 3 is integrally equipped with the syringe 10a for collecting concentrated adipose-derived cells. However, instead of this, as shown in FIG. 8, it is also possible to employ a kind of centrifuge container 10 having a cell suction tube 20 for sucking concentrated adipose-derived cells. The sucked adipose-derived cells may be driven by the pump 15 to be sent to a cell collection container (not shown). In the drawing, the reference sign 21 denotes a suction end for sucking the supernatant.

Moreover, an adipose tissue is used as an example of the biological tissue for isolating cells. However, the tissue is not to be limited to the adipose tissue, and this invention is also applicable to isolate cells originated from a different kind of biological tissue from the concerned biological tissue.

## Claims

1. A cell processing device comprising:
a plurality of processing containers for processing cells;
a processing liquid container for containing a processing liquid to be supplied to the processing containers;
a waste liquid container for containing a waste liquid which has been discharged from the processing containers after processing the cells in the processing containers;
a plurality of pipe lines for connecting these containers; a pump for supplying the processing liquid and the waste liquid via the pipe lines;
weight meters for weighing the processing containers, the waste liquid container, and/or the processing liquid container; and
a leakage determination section for determining the occurrence of a leakage of the processing liquid or the waste liquid based on outputs from the weight meters.

2. A cell processing device according to claim 1, wherein said leakage determination section determines the occurrence of a leakage by comparing a difference in outputs from said weight meters between before and after the waste liquid has been discharged from said processing containers, with a difference in outputs from said weight meters between before and after the waste liquid has flown into said waste liquid container.

3. A cell processing device according to claim 1, wherein said leakage determination section determines the occurrence of a leakage by comparing a difference in outputs from said weight meters between before and after the processing liquid has been supplied from said processing liquid container, with a difference in outputs from said weight meters between before and after the waste liquid has flown into said waste liquid container.
